# EUROPEAN PATENT SPECIFICATION

(11) **EP 3 531 132 B1**
(45) Date of publication and mention of the grant of the patent: **10.08.2022**
(21) Application number: 19158349.1
(22) Date of filing: 20.02.2019
(51) Int. Cl.: G01N 33/543, G01N 33/58

(54) **KIT FOR MEASURING SUBSTANCE TO BE MEASURED, FLUORESCENT LABELING AGENT, AND FLUORESCENTLY LABELED ANTIBODY**
KIT ZUM MESSEN EINER ZU MESSENDEN SUBSTANZ, FLUORESZENTES MARKIERUNGSMITTEL UND FLUORESZENT MARKIERTER ANTIKÖRPER
KIT POUR MESURER UNE SUBSTANCE A MESURER, AGENT D'ETIQUETAGE FLUORESCENT ET ANTICORPS ÉTIQUETÉ DE FAÇON FLUORESCENTE

(30) Priority: 22.02.2018 JP 2018029327
(43) Date of publication of application: 28.08.2019
(73) Proprietor: FUJIFILM Corporation, Tokyo 106-8620 (JP)
(72) Inventor: KANAZAWA, Yoshinori, Kanagawa 258-8538 (JP); CHIKU, Hiroyuki, Kanagawa 258-8538 (JP)
(74) Representative: Klunker IP Patentanwälte PartG mbB

(56) References cited:
- EP-A1- 3 505 592
- WO-A1-2016/122117
- US-A1- 2012 009 615
- CESAR F. A. GÓMEZ-DURÁN ET AL: "Near-IR BODIPY Dyes à la Carte-Programmed Orthogonal Functionalization of Rationally Designed Building Blocks", CHEMISTRY - A EUROPEAN JOURNAL, vol. 22, no. 3, 26 November 2015 (2015-11-26), pages 1048-1061, XP055603178, DE ISSN: 0947-6539, DOI: 10.1002/chem.201503090
- ZHU SHILEI ET AL: "Highly water-soluble, near-infrared emissive BODIPY polymeric dye bearing RGD peptide residues for cancer imaging", ANALYTICA CHIMICA ACTA, vol. 758, 1 November 2012 (2012-11-01), pages 138-144, XP028961245, ISSN: 0003-2670, DOI: 10.1016/J.ACA.2012.10.026
- SHILEI ZHU ET AL: "Highly water-soluble neutral near-infrared emissive BODIPY polymeric dyes", JOURNAL OF MATERIALS CHEMISTRY, vol. 22, no. 6, 1 January 2012 (2012-01-01), pages 2781-2790, XP055603185, GB ISSN: 0959-9428, DOI: 10.1039/C2JM14920F

## Description

### BACKGROUND OF THE INVENTION

### 1. Field of the Invention

The present invention relates to a kit for measuring a substance to be measured which is formed of a luminescent compound having a specific structure. The present invention further relates to a fluorescent labeling agent.

### 2. Description of the Related Art

A fluorescence detection method has been in wide use as a highly sensitive and simple measurement method for quantitatively determining proteins, enzymes, inorganic compounds, and the like. The fluorescence detection method is a method in which fluorescence emitted in a case where a sample considered to include a substance to be measured which is excited by light of a specific wavelength so as to emit fluorescence, is irradiated with excitation light of the specific wavelength, is detected, and thus the presence of the substance to be measured is confirmed. In a case where the substance to be measured is not a fluorescent substance, for example, a substance that specifically binds to the substance to be measured is brought into contact with a sample while the substance is in a state of being labeled with a fluorescent dye, and thereafter, in the same manner as above, fluorescence emitted in a case where the sample is irradiated with the excitation light is detected, and thus the presence of the substance to be measured can be confirmed.

In the fluorescence detection method as described above, in order to improve the sensitivity for detecting a small amount of a substance to be measured, a method utilizing the effect of electric field enhancement by plasmon resonance has been known. In this method, in order to generate plasmon resonance, a sensor chip including a metal layer provided in a predetermined region on a transparent support is prepared. The excitation light is allowed to enter an interface between the support and the metal film from a surface side opposite to a metal layer formation surface of the support at a predetermined angle equal to or greater than a total reflection angle. Surface plasmons are generated in the metal layer by irradiation with such excitation light. The fluorescence is enhanced by the electric field enhancement effect due to generation of the surface plasmons, leading to an increase of a signal/noise ratio (S/N ratio), and therefore measurement can be performed with high-sensitivity. The fluorescence detection method by surface plasmon excitation (hereinafter referred to as "SPF method") can obtain a degree of signal enhancement about 10 times compared to the fluorescence detection method by epi-excitation (also referred to as epifluorescence method), and thus can perform the measurement with high-sensitivity.

JP2012-513974A discloses a dipyrromethene-boron hydrophilic fluorescent compound. JP2012-513974A discloses that the above-described hydrophilic fluorescent compound is used as a fluorescent marker in an aqueous medium. JP2012-513973A discloses a fluorescent compound derived from dipyrromethene-boron which is not fluorescent. JP2012-513973A discloses that the above-described fluorescent compound is used for fluorescent labeling of a biological molecule. CESAR F.A. GOMEZ-DURAN ET AL: "Near-IR BODIPY Dyes a la Carte-Programmed Orthogonal Functionalization of Rationally Designed Building Blocks", CHEMISTRY - A EUROPEAN JOURNAL, vol. 22, no. 3, 26 November 2015 (2015-11-26), pages 1048-1061, discloses the synthesis of polyfunctional BODIPY building blocks suitable to be subjected to reaction sequences with complete chemoselectivity, thereby allowing the preparation of complex BODIPY derivatives in a versatile and programmable manner. The synthetic protocol allows to design compounds with tailored photophysical properties, i.e. emissions deep in the near-infrared spectral region in combination with high fluorescence quantum yields.

ZHU SHILEI ET AL: "Highly water-soluble, near-infrared emissive BODIPY polymeric dye bearing RGD peptide residues for cancer imaging", ANALYTICA CHIMICA ACTA, vol. 758, pages 138-144 discloses the preparation of near-infrared emissive BODIPY polymeric dye bearing bromide groups and of BODIPY polymeric dye bearing RGD peptides by post-polymerization functionalization.

ZHU SHILEI ET AL: "Highly water-soluble neutral, near-infrared emissive BODIPY polymeric dyes", JOURNAL OF MATERIALS CHEMISTRY, vol. 22, NO. 6, 1 January 2012 (2012-01-01), pages 2781-2790, discloses BODIPY polymeric dyes having hydrophilic groups and capture groups.

### SUMMARY OF THE INVENTION

The fluorescent compounds disclosed in JP2012-513974A and JP2012-513973A have problems of low quantum yield and low measurement sensitivity. An object of the present invention is to provide a kit for measuring a substance to be measured which is capable of high-sensitivity measurement. Another object of the present invention is to provide a fluorescent labeling agent which has an emission maximum wavelength within a long wavelength range of 680 nm or longer, and exhibits a high quantum yield.

As a result of extensive studies to solve the above problems, the inventors of the present invention have found that a kit for measuring a substance to be measured which is capable of high-sensitivity measurement can be provided by using a compound having a specific structure which has an emission maximum wavelength within a long wavelength range of 680 nm or longer, and exhibits a high quantum yield as a fluorescent labeling agent, and therefore have completed the present invention.

Accordingly, subject-matter of the present invention is a kit for measuring a substance to be measured, the kit having the features indicated in claim 1, and a fluorescent labeling agent having the features indicated in claim 6. Embodiments of the invention are indicated in the respective dependent claims.

According to the kit for measuring a substance to be measured according to the present invention, high-sensitivity measurement is possible. The fluorescent labeling agent of the present invention has an emission maximum wavelength within a long wavelength range of 680 nm or longer, and exhibits a high quantum yield.

### BRIEF DESCRIPTION OF THE DRAWINGS

Fig. 1 shows a schematic view of a sensor chip.
Fig. 2 shows an exploded view of the sensor chip.

### DESCRIPTION OF THE PREFERRED EMBODIMENTS

Hereinafter, embodiments of the present invention will be described in detail.

In the present specification, a range of numerical values described using "to" means a range including the numerical values listed before and after "to" as a minimum value and a maximum value.

The present invention relates to a kit for measuring a substance to be measured which includes a compound represented by Formula (1) that binds to a first binding substance capable of binding to the substance to be measured; and a substrate that includes, on a metal film, a detection region having a second binding substance capable of binding to any one of the substance to be measured or the first binding substance.

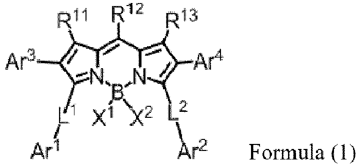

The meaning of each symbol in Formula (1) is as defined in the present specification.

In general, in fluorescent dyes hydrophilized with hydrophilic groups, the quantum yield (sensitivity) decreases due to aggregation by a hydrophobic skeleton in an aqueous system. In the compound represented by Formula (1) used in the present invention, Ar³ and Ar⁴ are orthogonal to the dipyrromethene skeleton, and have a specific structure where an interaction between the molecules is lowered, thereby suppressing aggregate quenching, which is presumed to lead to an improvement in sensitivity.

In the present specification, an alkyl group may be any of a linear, branched, or cyclic alkyl group, or a combination thereof, and the number of carbon atoms of the linear or branched alkyl group is preferably from 1 to 36, more preferably from 1 to 18, even more preferably from 1 to 12, and particularly preferably from 1 to 6. Examples of the cyclic alkyl group include cycloalkyl having 3 to 8 carbon atoms. Specific examples of the alkyl group include a methyl group, an ethyl group, an n-propyl group, an isopropyl group, an n-butyl group, an iso-butyl group, a sec-butyl group, a t-butyl group, an n-pentyl group, an n-hexyl group, an n-heptyl group, an n-octyl group, an n-nonyl group, an n-decyl group, an n-undecyl group, an n-dodecyl group, an n-tridecyl group, an n-tetradecyl group, an n-pentadecyl group, an n-hexadecyl group, an n-heptadecyl group, an n-octadecyl group, and a cyclohexyl group.

In the present specification, as an aryl group, an aryl group having 6 to 48 carbon atoms is preferable, an aryl group having 6 to 24 carbon atoms is more preferable, and an aryl group having 6 to 14 carbon atoms is even more preferable. Examples thereof include a phenyl group, a naphthyl group, an anthryl group, a pyrenyl group, a phenanthrenyl group, a biphenyl group, and a fluorenyl group.

In the present specification, an acyl group is preferably a linear or branched alkanoyl group having 2 to 15 carbon atoms, and examples thereof include an acetyl group, a propionyl group, a butyryl group, an isobutyryl group, a valeryl group, an isovaleryl group, a pivaloyl group, a hexanoyl group, a heptanoyl group, and a benzoyl group.

In the present specification, an alkoxy group is preferably an alkoxy group having 1 to 20 carbon atoms, and examples thereof include a methoxy group, an ethoxy group, a propoxy group, an n-butoxy group, a pentyloxy group, a hexyloxy group, and a heptyloxy group.

In the present specification, the aryloxy group is preferably an aryloxy group having 6 to 14 carbon atoms, and examples thereof include a phenoxy group, a naphthoxy group, and an anthryloxy group.

An alkylthio group is preferably an alkylthio group having 1 to 30 carbon atoms, and examples thereof include a methylthio group, an ethylthio group, and an n-hexadecylthio group.

The arylthio group is preferably an arylthio group having 6 to 30 carbon atoms, and examples thereof include a phenylthio group, a p-chlorophenylthio group, and an m-methoxyphenylthio group.

In the present specification, examples of a halogen atom include a fluorine atom, a chlorine atom, a bromine atom, and an iodine atom.

In the present specification, examples of an aromatic ring include an aromatic hydrocarbon ring such as a benzene ring, a naphthalene ring, an anthracene ring, a phenanthrene ring, a pyrene ring, a perylene ring, and a terylene ring; an aromatic heterocyclic ring such as an indene ring, an azulene ring, a pyridine ring, a pyrazine ring, a pyrimidine ring, a pyrazole ring, a pyrazolidine ring, a thiazolidine ring, an oxazolidine ring, a pyran ring, a chromene ring, a pyrrole ring, a pyrrolidine ring, a benzimidazole ring, an imidazoline ring, an imidazolidine ring, an imidazole ring, a pyrazole ring, a triazole ring, a triazine ring, a diazole ring, an indoline ring, a thiophene ring, a thienothiophene ring, a furan ring, an oxazole ring, an oxadiazole ring, a thiazine ring, a thiazole ring, an indole ring, a benzothiazole ring, a benzothiadiazole ring, a naphthothiazole ring, a benzoxazole ring, a naphthoxazole ring, an indolenine ring, a benzindolenine ring, a pyrazine ring, a quinoline ring, and a quinazoline ring; a fused aromatic ring such as a fluorene ring and a carbazole ring. An aromatic ring having 5 to 16 carbon atoms (an aromatic ring and a condensed ring containing an aromatic ring) is preferable.

The aromatic ring may have a substituent, and the term "aromatic ring" means both an aromatic ring having a substituent and an aromatic ring having no substituent. Examples of the substituent that the aromatic ring has include substituents to be described in the section of Substituent group A.

Examples of an amino group include an amino group; an alkyl-substituted amino group such as mono- or dimethylamino group, mono- or diethylamino group, and mono- or di-(n-propyl) amino group; an amino group substituted with an aromatic residue such as mono- or diphenylamino group and mono- or dinaphthylamino group; an amino group in which an alkyl group and an aromatic residue are substituted one by one, such as a monoalkyl monophenylamino group; a benzylamino group, an acetylamino group, and a phenylacetyl amino group. The aromatic residue referred herein means a group in which one hydrogen atom has been removed from an aromatic ring, and the aromatic ring is as described above in the present specification.

The alkyl group represented by R¹¹ and R¹³ and the phenyl group represented by R¹² each may have a substituent, and the substituents are selected from substituents of the following substituent group A. The substituent of the substituent group A may be further substituted by a substituent of the substituent group A.

### Substituent group A:

A sulfamoyl group, a cyano group, an isocyano group, a thiocyanato group, an isothiocyanato group, a nitro group, a nitrosyl group, a halogen atom, a hydroxy group, an amino group, a mercapto group, an amide group, an alkoxy group, an aryloxy group, an alkylthio group, an arylthio group, a carbamoyl group, an acyl group, an aldehyde group, a carbonyl group, an aryl group, an alkyl group, an alkyl group substituted with a halogen atom, an ethenyl group, an ethynyl group, a silyl group, and a trialkylsilyl group (such as a trimethylsilyl group).

The alkoxy group represented by X¹ and X² may have a substituent, and examples of the substituent include the substituents described in Substituent group A.

The aryl group represented by Ar¹, Ar², Ar³, and Ar⁴ may have a substituent, and examples of the substituent include the substituents described in Substituent group A.

At least one of R¹¹, R¹², R¹³, Ar¹, Ar², Ar³, Ar⁴, X¹, or X² has a hydrophilic part, at least one of R¹¹, R¹², R¹³, Ar¹, Ar², Ar³, Ar⁴, X¹, or X² has a capture part capable of forming a covalent bond with a biological molecule.

The hydrophilic part has a polyethylene glycol structure, a betaine structure, an amino acid structure, or a sulfonic acid group.

Examples of the polyethylene glycol structure include a structure represented by -(CH₂-CH₂-O)ₙ-H (in the formula, n represents an integer of 1 to 10).

As the betaine structure, a betaine structure coexisting at a position where an anionic ion and a cationic ion are not adjacent to each other is preferable. An anionic ion portion and a cationic ion portion are chemically bonded through a linking group. Examples of the anionic ion portion include carboxylate ion (-COO⁻), sulfate ion (-OSO₃⁻), sulfonate ion (-SO₃⁻), and phosphate ion (-OPO₃⁻-). Examples of the cationic ion portion include a quaternary ammonium ion and the like. In addition, examples of the linking group include an alkylene group having 1 to 6 carbon atoms.

Examples of the amino acid structure include a structure derived from amino acids (such as alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine), that is, a structure in which a carboxy group and an amino group are bonded to a carbon atom.

The capture part capable of forming the covalent bond with the biological molecule is a carboxyl group, an active ester, an isothiocyanate, or a maleimide.

Examples of the active ester include a chemical structure of -C(=O)-X. X means a leaving group such as halogen, N-hydroxysuccinimide group or a derivative thereof, 1-hydroxybenzotriazole group or a derivative thereof, pentafluorophenyl group, paranitrophenyl group, and the like, but X is not limited thereto. The active ester is preferably N-hydroxysuccinimide ester.

### Compound represented by Formula (1)

In Formula (1), R¹¹ and R¹³ each independently represent an alkyl group, and these groups may have a substituent.

R¹¹ and R¹³ preferably represent a methyl group.

R¹² represents a phenyl group that may have a substituent.

R¹² preferably represents a phenyl group having the capture part capable of forming the covalent bond with the biological molecule (a carboxyl group, an active ester, an isothiocyanate, or a maleimide), or a phenyl group substituted with a fluorine atom.

In Formula (1), L¹ and L² each represent Formula (L-1.

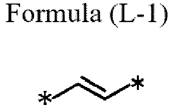

In Formula (1), Ar¹, Ar², Ar³, and Ar⁴ each independently represent an aryl group, and these groups may have a substituent.

At least one of Ar¹ or Ar² in Formula (1) is a group represented by Formula (Ar-1), and preferably, Ar¹ and Ar² in Formula (1) are the group represented by Formula (Ar-1).

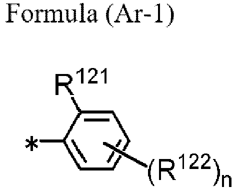

In the formula, R¹²¹ and R¹²² each independently represent a halogen atom, an alkyl group, or an alkoxy group, provided that R¹²¹ and R¹²² each may have the hydrophilic part or the capture part capable of forming the covalent bond with the biological molecule. n represents an integer of 0 to 4, and in a case where n is 2 or more, a plurality of R¹²²' s may be the same or different from each other.

At least one of Ar³ or Ar⁴ in Formula (1) is a group represented by Formula (Ar-1), and preferably, Ar³ and Ar⁴ in Formula (1) are the group represented by Formula (Ar-1).

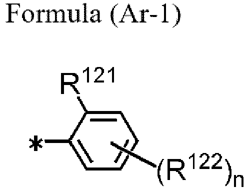

In the formula, R¹²¹ and R¹²² each independently represent a halogen atom, an alkyl group, or an alkoxy group, provided that R¹²¹ and R¹²² each may have the hydrophilic part or the capture part capable of forming the covalent bond with the biological molecule. n represents an integer of 0 to 4, and in a case where n is 2 or more, a plurality of R¹²²' s may be the same or different from each other.

In Formula (1), X¹ and X² each independently represent a halogen atom or an alkoxy group, and these groups may have a substituent.

More preferably, both X¹ and X² are halogen atoms, or both X¹ and X² represent alkoxy groups substituted with a group having a polyethylene glycol structure.

In Formula (1), at least one of R¹¹, R¹², R¹³, Ar¹, Ar², Ar³, Ar⁴, X¹, or X² has the hydrophilic part, at least one of R¹¹, R¹², R¹³, Ar¹, Ar², Ar³, Ar⁴, X¹, or X² has the capture part capable of forming the covalent bond with the biological molecule.

### Specific example of compound represented by Formula (1)

A specific example of the compound represented by Formula (1) is described below.

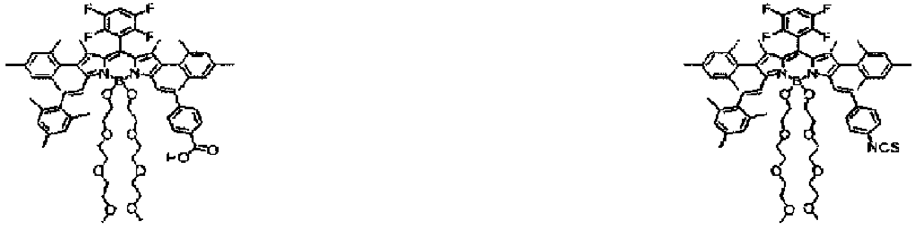

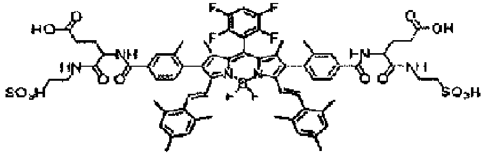

In the present invention, at least one kind of the compound represented by Formula (1) is used, but two or more kinds of the compound represented by Formula (1) may be combined to be used.

### Production method of compound represented by Formula (1)

The compound represented by Formula (1) can be produced by, for example, a synthesize scheme shown in examples to be described later.

As an example, the outline of the synthesis of the reference compound (1) (a structure thereof is described in the examples described later) is shown below. Ethyl terephthalaldehyde and 2,4-dimethylpyrrole are mixed in dichloromethane, and next, trifluoroacetic acid and chloranil are added to the mixture, followed by stirring, and then diisopropylethylamine is added. After stirring the mixture, boron trifluoride diethyl ether complex is added and stirred. Therefore, a compound (1-A) (a structure thereof is described in the examples described later) is obtained.

The compound (1-A) and 1,1,1,3,3,3-hexafluoro-2-propanol are mixed, N-iodosuccinimide (NIS) is added to the mixture, sodium thiosulfate is added thereto, and therefore a compound (1-B) (a structure thereof is described in the examples described later) is obtained.

Piperidine and p-toluenesulfonic acid are added to and allowed to react with the mixture of the compound (1-B), 2,4,6-trimethylbenzaldehyde, and toluene, and therefore a compound (1-C) (a structure thereof is described in the examples described later) is obtained.

SPhos Pd G3 is added to the mixture of the compound (1-C), 4-boronobenzenesulfonic acid, cesium fluoride, and methoxycyclopentane, the resultant mixture is then heated under reflux. Thereafter, a hydrochloric acid aqueous solution and ethyl acetate are added, the obtained reaction solution is purified, and then a sodium hydroxide solution is added, and therefore a compound (1-D) (a structure thereof is described in the examples described later) is obtained.

Water, EDC (1-[3-(dimethylamino) propyl]-3-ethylcarbodiimide) and N-hydroxysuccinimide are added to the compound (1-D) and stirred, and therefore the reference compound (1) can be obtained.

As another example, a compound (2) according to the invention can be produced by going through procedures of producing a compound (2-A), a compound (2-B), a compound (2-C), and a compound (2-D) according to a synthesis scheme of <Synthesis Example 2> in the example to be described later.

The compound (2) is within the definition of the compound represented by Formula (1). Compounds represented by Formula (1) other than the reference compound (1) and the compound (2) according to the invention can be produced by using a compound having a substituent corresponding to a desired compound represented by Formula (1) in place of the compound used in the reaction.

### Emission maximum wavelength and quantum yield of compound represented by Formula (1)

The emission maximum wavelength represents a wavelength at which an absorbance becomes the largest in an absorption spectrum.

The emission maximum wavelength of the compound represented by Formula (1) is preferably 600 nm or longer, more preferably 640 nm or longer, even more preferably 660 nm or longer, particularly preferably 680 nm or longer, and most preferably Is 700 nm or longer.

An upper limit of the emission maximum wavelength of the compound represented by Formula (1) is not particularly limited, and is preferably 900 nm or shorter, more preferably 800 nm or shorter.

The emission maximum wavelength of the compound can be measured using a commercially available fluorescence spectrophotometer, and can be measured by using, for example, fluorescence spectrophotometer RF-5300 PC manufactured by Shimadzu Corporation, absolute PL (photoluminescence) quantum yield measuring device C9920-02 manufactured by Hamamatsu Photonics KK.

The quantum yield is a ratio of the number of photons emitted as fluorescence to the number of photons absorbed by the compound.

The quantum yield of the compound represented by Formula (1) is preferably 0.65 or more, more preferably 0.7 or more, even more preferably 0.75 or more, still even more preferably is 0.8 or more, and particularly preferably 0.85 or more. An upper limit of the quantum yield is not particularly limited, but is generally 1.0 or less.

The quantum yield of the compound represented by Formula (1) can be measured using a commercially available quantum yield measuring device, and can be measured by using, for example, absolute PL (photoluminescence) quantum yield measuring device C9920-02 manufactured by Hamamatsu Photonics KK.

### Substance to be measured

The kit according to the embodiment of the invention is a kit for measuring a substance to be measured.

The substance to be measured may be a substance present in a biological sample. The biological sample is not particularly limited as long as the biological sample is a sample which may contain the substance to be measured, and examples thereof include a biological sample, particularly body fluids (for example, blood, blood serum, blood plasma, cerebrospinal fluid, tear fluid, sweat, urine, pus, nasal discharge, or expectoration) of animals (for example, humans, dogs, cats), excretas (for example, feces), organs, tissues, membrana mucosa, and skin.

The substance to be measured s not particularly limited, and examples thereof include cholesterol, hormone (such as steroid hormone, peptide hormone), protein, bile acid, and cortisol. Particularly preferable examples of the substance to be measured include progesterone or thyroid stimulating hormone (TSH).

### First binding substance

The first binding substance used in the present invention is a binding substance capable of binding to the substance to be measured. As the first binding substance, an antigen, an antibody, or a complex thereof can be used, but the first binding substance is not limited thereto. The first binding substance is preferably an antibody. In a case where the first binding substances are the antibodies, as the antibodies capable of binding to the substance to be measured, for example, an antiserum prepared from blood serum of an animal immunized with the substance to be measured, an immunoglobulin fraction purified from an antiserum, a monoclonal antibody obtained by cell fusion using spleen cells of an animal immunized with the substance to be measured, and fragments thereof [for example, F(ab')₂, Fab, Fab', or Fv] can be used. The preparation of these antibodies can be carried out by using a general method. Furthermore, an antibody modified as a case of e.g. a chimeric antibody may be used, or a commercially available antibody also may be used as long as the antibody is an antibody prepared from blood serum of an animal or culture supernatant by a known method.

### Compound represented by Formula (1) that binds to first binding substance capable of binding to substance to be measured

By allowing the capture part capable of forming the covalent bond with the biological molecule in the compound represented by Formula (1) to react with the first binding substance (for example, antibody), the first binding substance can bind to the compound represented by Formula (1).

Examples of a method for binding include a method in which the carboxyl group in the compound represented by Formula (1) is active-esterified by a water-soluble carbodiimide [WSC] (such as 1-ethyl-3-(3-dimethylaminopropyl) carbodiimide hydrochloride [EDC]) and N-hydroxysuccinimide [NHS], and then the active-esterified carboxyl group and an amino group of the first binding substance (e.g. antibody) are dehydrated using water-soluble carbodiimide so as to be immobilized. Alternatively, examples of a method for binding include a method in which the first binding substances (such as an antibody) each having an isothiocyanate and an amino group are reacted with the compound represented by Formula (1) so as to be immobilized; a method in which the first binding substances (such as an antibody) each having a sulfonyl halide and an amino group are reacted with the compound represented by Formula (1) so as to be immobilized; and a method in which the first binding substances (such as an antibody) each having iodoacetamide and a thiol group are reacted with the compound represented by Formula (1) so as to be immobilized; a method in which a biotinylated compound represented by Formula (1) is reacted with a streptavidinated first binding substance (such as antibody) (alternatively, a streptavidinated compound represented by Formula (1) and a biotinylated first binding substance (such as an antibody)) so as to be immobilized.

The compound represented by Formula (1) bound to the first binding substance capable of binding to the substance to be measured is contained in the kit according to the embodiment of the invention, but an aspect in which the compound is contained in a container which is a part of the kit, for example, a cup, is preferable. In this case, the biological sample is injected into the container, mixed, and stirred, whereby the first binding substance and the substance to be measured in the biological sample can bind to each other.

### Fluorescent labeling agent labeling an antibody

As described above, the compound represented by Formula (1) can be used by being bound to the first binding substance such as an antibody as a fluorescent label, and the compound represented by Formula (1) is useful as a fluorescent labeling agent. That is, according to the present invention, the fluorescent labeling agent containing at least one kind of the compounds represented by Formula (1) is provided.

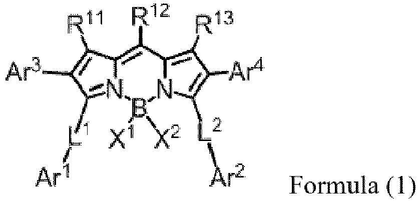

The meaning of each symbol in Formula (1) is as defined in the present specification.

According to the present invention, the above-described fluorescent labeling agent may be used for fluorescently labeling an antibody. The antibody is preferably bound to the fluorescent labeling agent via an amide bond.

### Substrate

In the present invention, in order to achieve highly sensitive measurement, it is preferable to adopt a measurement method that performs surface plasmon fluorescence (SPF) detection to be described below. As a substrate a substrate having a metal film on a surface thereof is used. A metal constituting the metal film is not particularly limited as long as it is a metal by which surface plasmon resonance can occur. Preferable examples thereof include free electron metals such as gold, silver, copper, aluminum, or platinum, and gold is particularly preferable. In a case where gold is used, the detection region to be described below is on a gold film. These metals can be used alone or in combination. Furthermore, in consideration of adhesion to the substrate, an intermediacy layer made of chromium or the like may be provided between the substrate and a layer formed of the metal. Any thickness of the metal film may be used, but is preferably, for example, 1 nm to 500 nm, and particularly preferably 10 nm to 200 nm. In a case where the thickness exceeds 500 nm, the surface plasmon phenomenon of a medium cannot be sufficiently detected. In addition, in a case of providing the intermediacy layer made of chromium or the like, the thickness of the intermediacy layer is preferably 0.1 nm to 10 nm.

The formation of the metal film may be carried out by a general method and can be carried out by, for example, a sputtering method, a vapor deposition method, an ion plating method, an electroplating method, or an electroless plating method, but in order to improve the adhesiveness of the metal film by providing a mix layer of the substrate material and the metal film, it is preferable to produce the metal film by the sputtering method. In this case, a thickness of the mix layer of the substrate material and the metal film is not particularly limited as long as sufficient adhesiveness can be secured, but is preferably 10 nm or less.

The metal film is preferably disposed on the substrate. The term "disposed on the substrate" means not only that the metal film is disposed in direct contact with the substrate, but also that the metal film is disposed via other layers without in direct contact with the substrate. As a material of the substrate which can be used in the present invention, for example, optical glass such as BK7 (borosilicate glass) which is one of general optical glasses, or synthetic resin, specifically, polymethyl methacrylate, polyethylene terephthalate, polycarbonate, or cycloolefin polymer, which is transparent to laser light can be used. It is preferable that such a substrate has a material which does not exhibit anisotropy with respect to polarized light and has excellent workability.

As a preferred aspect of the substrate for detecting SPF, there is a substrate obtained by vapor-depositing a gold film on polymethyl methacrylate (PMMA).

The substrate has, on a metal film, a detection region having a second binding substance capable of binding to any one of the substance to be measured and the first binding substance.

### Second binding substance

The second binding substance is a substance capable of binding to the substance to be measured or is a substance capable of binding to the first binding substance. In a case where the quantitative determination is carried out by a sandwich assay method, the substance capable of binding to the substance to be measured can be used as the second binding substance. In a case where the quantitative determination is carried out by a competitive method, the substance capable of binding to the first binding substance can be used as the second binding substance.

The second binding substance is not particularly limited, but preferable examples thereof include an antigen, an antibody, or a complex thereof. In a case where an antigen is used as the second binding substance, it is preferable to use the substance to be measured (which is the substance capable of binding to the first binding substance) or a hapten thereof. In a case where the substance to be measured or the hapten thereof is used as the second binding substance, the second binding substance is preferably a conjugate of the substance to be measured and a carrier. The term "carrier" means a substance to which a plurality of molecules of the substance to be measured can bind. Preferable examples of the carrier include proteins and the like, and specific examples include bovine serum albumin.

In a case of using an antibody as the second binding substance, as a method of immobilizing the antibody on the metal film on the substrate, it is possible to adopt any principle of physical adsorption or chemical bond by covalent bond. In addition, for the purpose of preventing metal quenching, it is possible to adopt a method of forming a self-assembled monolayer (SAM) on the metal film and immobilizing the second binding substance on the SAM film, or a method in which a hydrophilic polymer layer such as carboxymethyl dextran (CMD) is provided on the SAM and the second binding substance is immobilized to the hydrophilic polymer layer.

The self-assembled monolayer (SAM) is typically known as a monomolecular film with high orientation, which is formed on a gold surface due to the interaction between long alkyl chains, and forming of an Au-S bond at the time where an alkanethiol compound is brought into contact with a surface of the metal film containing gold, and is allowed to stand, and the surface containing gold reacts with the alkanethiol compound. The SAM is widely used in fields such as surface plasmon resonance/fluorescence and quartz crystal microbalance (QCM).

Specific examples of the SAM include a film composed of 1-undecanethiol, 10-carboxy-1-decanethiol, 11-hydroxy-1-undecanethiol, and a film of a combination thereof.

It is preferable to provide the hydrophilic polymer layer on the SAM, and this hydrophilic polymer layer is provided for forming a two-dimensional structure or a three-dimensional structure on the SAM In particular, in a case of forming the three-dimensional structure, the immobilization of the second binding substance is not limited to the two dimensions of the support surface, and it is possible to produce the structure of the hydrophilic polymer layer which is spread out to the three dimensional space liberated from the surface of the metal film on the substrate.

Specific examples of this hydrophilic polymer include at least one polymer selected from the group consisting of polysaccharide, polyethylene glycol, polyacrylic acid, and polymethacrylic acid. The polysaccharide is preferably a hydrophilic polymer such as dextran or a dextran derivative, and is preferably a hydrophilic polymer layer composed of dextran such as carboxymethyl dextran (CMD) from the viewpoint of improving biocompatibility or improving inhibitory properties of a nonspecific adsorption reaction.

In the case of using an antibody as the second binding substance, examples of a method of immobilizing on this hydrophilic polymer layer include a method in which a carboxyl group of a polymer having a reactive functional group such as carboxymethyl dextran (CMD) is active-esterified by a water-soluble carbodiimide (WSC) (such as 1-ethyl-3-(3-dimethylaminopropyl) carbodiimide hydrochloride (EDC)) and N-hydroxysuccinimide (NHS), and then the active-esterified carboxyl group and an amino group of the antibody that is the second binding substance are dehydrated using water-soluble carbodiimide so as to be immobilized.

### Detection region (test area)

In the present invention, the detection region (test area) for detecting the presence or absence of the substance to be measured is provided on the substrate. In this detection region, it is possible to quantitatively determine antigens by, for example, capturing the antigens which are the substances to be measured and detecting and quantitatively determining an amount of label bound to the antigens. Alternatively, it is possible to quantitatively determine the antigens by a method of preventing only a label bound to the antigens from binding, capturing only a label not bound to the antigens, and calculating an amount of label bound to the antigens.

### Antibody

In the present invention, the antibody can be used regardless of animal species or subclass thereof. For example, the antibody that can be used in the present invention is an antibody derived from an organism in which an immunologic response can occur, such as mouse, rat, hamster, goat, rabbit, sheep, cow, and chicken. Specific examples thereof include mouse IgG, mouse IgM, rat IgG, rat IgM, hamster IgG, hamster IgM, rabbit IgG, rabbit IgM, goat IgG, goat IgM, sheep IgG, sheep IgM, bovine IgG, bovine IgM, and avian IgY, and both polyclonal and monoclonal antibodies can be used. Furthermore, fragmented antibodies may be used. Fragmented antibodies are molecules derived from intact antibodies having at least one antigen binding site, and specific examples thereof include Fab, and F(ab')₂. These fragmented antibodies are molecules obtained by enzymatic or chemical treatment or using genetic engineering techniques.

### Other components of kit

The kit of the present invention is used for the method for measuring the substances to be measured, is a kit for diagnosis of the progesterone measurement in a case where the substances to be measured are the progesterone, and is a kit for diagnosis of the thyroid stimulating hormone (TSH) measurement in a case where the substances to be measured are TSH. In a case of carrying out the measurement of the substances to be measured, the kit according to the embodiment of the invention contains the compound represented by Formula (1) bound to the first binding substance capable of binding to the substance to be measured, and the substrate having, on the metal film, the detection region having the second binding substance, but various devices or instruments used for measuring the substances to be measured such as surface plasmon excitation devices and fluorescence measuring devices may be provided. Furthermore, as a component of the kit, e.g. a sample containing substances to be measured of known amounts, and an instruction manual may be included.

### Competitive method

As an example of the competitive method, a case of quantitatively determining progesterone is described below. A case of quantitatively determining substances other than progesterone, can also be carried out in the same manner.

First, a progesterone-containing biological sample and a fluorescently labeled anti-progesterone antibody are brought into contact with the progesterone immunoassay substrate on which the progesterone-albumin conjugate is immobilized. In a case where progesterone is not present in the biological sample, antigen-antibody reaction occurs on the substrate by the fluorescently labeled anti-progesterone antibody and progesterone on the substrate (that is, progesterone in the progesterone-albumin conjugate). Meanwhile, in a case where progesterone is present in the biological sample, an antigen-antibody reaction occurs between progesterone in the biological sample and the fluorescently labeled anti-progesterone antibody, and the antigen-antibody reaction is inhibited between the fluorescently labeled anti-progesterone antibody and progesterone on the substrate (that is, progesterone in the progesterone-albumin conjugate). After the above reaction is completed, the fluorescently labeled anti-progesterone antibody not bound to albumin on the substrate is removed. Next, a degree of formation of immune complexes on the substrate (that is, a complex of fluorescently labeled anti-progesterone antibody and progesterone in the progesterone-albumin conjugate on the substrate) is detected as fluorescence intensity. Accordingly, a concentration of progesterone in the biological sample can be measured.

As a measurement form of fluorescence in the competitive method, it is possible to adopt any one of plate reader measurement and flow measurement, and for example, the measurement can be carried out by the following method. A plurality of samples with known amount of progesterone, each sample having different progesterone concentrations, are prepared in advance, and this sample and the fluorescently labeled anti-progesterone antibody are mixed in advance. This mixed solution is brought into contact with a region on which the progesterone-albumin conjugate is immobilized. A fluorescent signal from the region on which the progesterone-albumin conjugate is immobilized is measured as a plurality of fluorescent signals while the mixed solution is in contact with the conjugate at specific time intervals. Based on the plurality of fluorescent signals, a time change (inclination) of the amount of fluorescence is obtained at each progesterone concentration. By plotting this time change as a Y-axis and the progesterone concentration as an X-axis, a relational expression of the progesterone concentration with respect to the time change of the amount of fluorescence is acquired by using an appropriate fitting method such as a least squares method or the like. Based on the relational expression thus obtained, the amount of progesterone contained in the biological sample can be quantitatively determined by using the result of the time change of the amount of fluorescence using the biological sample to be examined.

The quantitative determination of the amount of progesterone is preferably carried out in a short period of time. Specifically, the quantitative determination is preferably carried out within 10 minutes, more preferably within 8 minutes, and even more preferably within 6 minutes. By using the relational expression between the time change of the amount of fluorescence and the progesterone concentration acquired in advance using an appropriate fitting method such as the least squares method, the sample and the fluorescently labeled anti-progesterone antibody are brought into contact with the detection region to which the progesterone-albumin conjugate is immobilized, and then a time for converting the amount of progesterone contained in the biological sample is obtained based on the result of the time change of the amount of fluorescence using the biological sample to be examined, and a time for the quantitative determination preferably includes this time for conversion.

### Sandwich method

As an example of the sandwich method, a case of quantitatively determining TSH is described below. A case of quantitatively determining substances other than TSH, can also be carried out in the same manner.

First, a TSH-containing biological sample and a fluorescently labeled anti-TSH antibody 2 are brought into contact with a TSH immunoassay substrate on which an anti-TSH monoclonal antibody 1 is immobilized. In a case where TSH is present in the biological sample, an antigen-antibody reaction occurs on the substrate by TSH which has undergone antigen-antibody reaction with the fluorescently labeled anti-TSH antibody 2 in advance, and the anti-TSH antibody 1 on the substrate. Meanwhile, in a case where TSH is not present in the biological sample, the antigen-antibody reaction does not occur between the fluorescently labeled anti-TSH antibody 2 mixed with the biological sample, and the anti-TSH antibody 1 on the substrate. After the above reaction is completed, the fluorescently labeled anti-TSH antibody 2 not bound to the anti-TSH antibody 1 on the substrate is removed. Next, a degree of formation of immune complexes on the substrate (that is, a complex of the anti-TSH antibody 1 on the substrate, and the fluorescently labeled anti-TSH antibody 2 and TSH) is detected as fluorescence intensity. Accordingly, a concentration of the substance to be measured can be measured. The fluorescence intensity and the concentration of substances to be measured have a positive correlation.

### Flow passage

In a preferable aspect a mixed solution obtained by mixing the biological sample that may contain the substances to be measured and the compound represented by Formula (1) bound to the first binding substance can be applied on the substrate and developed in a flow passage. The flow passage is not particularly limited as long as it is a passage that allows the biological sample and the compound represented by Formula (1) bound to the first binding substance to flow down to the detection region. Preferable aspects of the flow passage include a spotting port for spotting the biological sample solution containing the compound represented by Formula (1) bound to the first binding substance, the metal film as the detection region, and a flow passage beyond the metal film. The biological sample has a structure capable of passing over the metal film. Preferably, a suction port can be provided on the side opposite to the spotting port with respect to the metal film.

### Measurement of surface plasmon fluorescence

A method for detecting labels such as fluorescence in the present invention is not particularly limited, and examples thereof include a device capable of detecting fluorescence intensity. Specifically, it is preferable to detect fluorescence intensity using a microplate reader, or biosensor for performing fluorescence detection (SPF) by surface plasmon excitation. The label information related to an amount of the substances to be measured is preferably acquired from fluorescence detection by surface plasmon resonance.

The form of measurement of fluorescence may be plate reader measurement or flow measurement. The fluorescence detection method (SPF method) by surface plasmon excitation can perform the measurement with higher sensitivity than the fluorescence detection method (epifluorescence method) by epi-excitation.

As a surface plasmon fluorescence (SPF) biosensor, for example, it is possible to use a sensor, which is described in JP2008-249361A, the sensor including an optical waveguide formed from a material that transmits excitation light of a predetermined wavelength, a metal film formed on one surface of this optical waveguide, a light source for generating a light beam, an optical system that allows the light beam to pass through the optical waveguide and causes the light beam to enter the interface between the optical waveguide and the metal film at an incident angle that generates surface plasmons, and a fluorescence detection means for detecting the fluorescence generated by being excited by the above-described evanescent wave enhanced by the surface plasmon.

The fluorescence detection (SPF) system by surface plasmon excitation is a preferably an assay method for detecting fluorescence from the fluorescent substance dependent on the amount of substances to be measured immobilized on the metal film on the substrate, which is the method different from a so-called latex agglutination method in which a change in optical transparency is detected as, for example, turbidity due to progress of reaction in a solution. In the latex agglutination method, an antibody-sensitized latex in the latex reagent and the antigen in the biological sample bind and aggregate by an antibody reaction, but the agglomerate increases with time, and an antigen concentration is quantitatively determined from a change in absorbance per unit time obtained by irradiating the aggregate with near infrared light. It is possible to provide a significantly simple method for detecting the substances to be measured as compared with the latex agglutination method.

Hereinafter, the present invention will be more specifically described with reference to the examples of the present invention. Et represents ethyl in the following chemical formula.

### Examples

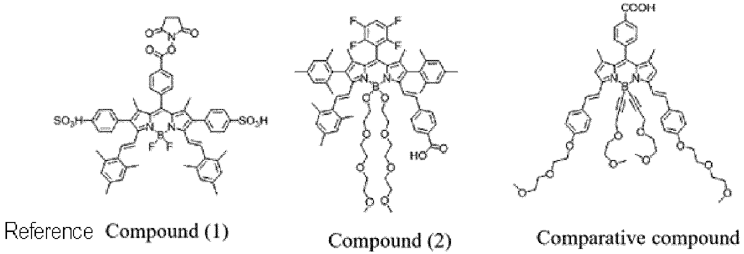

### Synthesis Example 1

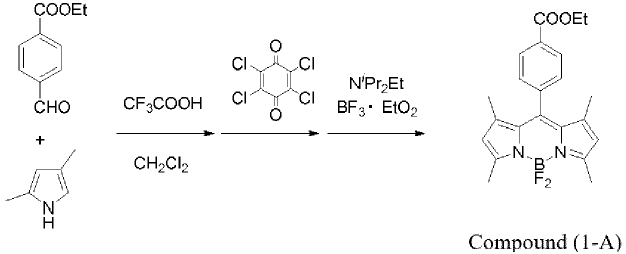

In a 100 mL three-necked flask, 1.07 g of ethyl terephthalaldehyde and 50 mL of dichloromethane were introduced under a nitrogen atmosphere, and the mixture was stirred at room temperature. 1.14 g of 2,4-dimethylpyrrole was added dropwise while cooling with water, followed by adding 5 drops of trifluoroacetic acid, and the resultant mixture was stirred at room temperature for 1 hour. While cooling with water, 2.94 g of chloranil was added, and after stirring at room temperature for 1 hour, 5.43 g of diisopropylethylamine (NⁱPr₂Et) was added dropwise while cooling with water, and the mixture was stirred at room temperature for 30 minutes. Subsequently, 8.29 mL of a boron trifluoride diethyl ether complex was added dropwise while cooling with water, and the mixture was stirred at room temperature for 1 hour. 100 ml of distilled water was added, and an organic layer obtained by extraction and liquid separation was preliminarily dried with anhydrous sodium sulfate, and then concentrated under reduced pressure. This crude product was purified by silica gel column chromatography (eluent: hexane/ethyl acetate) and then recrystallized from methanol to obtain 1.02 g of a compound (1-A). The compound (1-A) was identified by ESI-MS. The results are shown below.

ESI-MS: [M + H]⁺ = 397

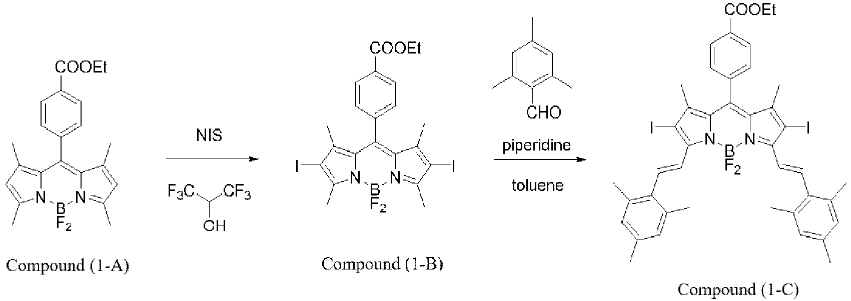

In a 300 mL three-necked flask, 2.63 g of the compound (1-A) and 60 mL of 1,1,1,3,3,3-hexafluoro-2-propanol were introduced and stirred at room temperature. 3.60 g of N-iodosuccinimide (NIS) was introduced, and the mixture was stirred at room temperature for 1.5 hours. After concentrating the reaction solution under reduced pressure, 50 mL of sodium thiosulfate aqueous solution (dissolved with 10 g of sodium thiosulfate) and 100 mL of methylene chloride were added, and an organic layer obtained by extraction and liquid separation was preliminarily dried with anhydrous sodium sulfate, and then concentrated under reduced pressure. This crude product was recrystallized from ethanol to obtain 3.27 g of a compound (1-B). The compound (1-B) was identified by ESI-MS. The results are shown below.

ESI-MS: [M + H]⁺ = 648

In a 100 mL flask, 0.37 g of the compound (1-B), 0.59 g of 2,4,6-trimethylbenzaldehyde, and 50 mL of toluene were added, 5 mL of piperidine and a small amount of para-toluenesulfonic acid were added thereto, and the resultant mixture was refluxed in an oil bath for 3 hours. The crude product obtained by concentrating the reaction solution under reduced pressure was purified by silica gel column chromatography (eluent: hexane/ethyl acetate) and then recrystallized from methanol to obtain 0.40 g of a compound (1-C). The compound (1-C) was identified by ESI-MS. The results are shown below.

ESI-MS: [M + H]⁺ = 909

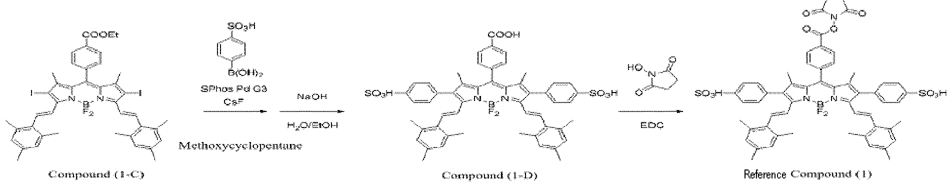

In a 100 mL three-necked flask, 500 mg of the compound (1-C), 1000 mg of 4-boronobenzenesulfonic acid, 1152.2 mg of cesium fluoride, and 26 mL of methoxycyclopentane were introduced, and the mixture was degassed under reduced pressure while stirring at room temperature, and therefore a nitrogen atmosphere was set. 322 mg of SPhos Pd G3 (manufactured by Aldrich) was added to the resultant mixture, and heated under reflux for 1 hour. 100 mL of 1 mol/L hydrochloric acid aqueous solution and 100 mL of ethyl acetate were added, the resulting reaction solution was concentrated under reduced pressure, and the crude product thus obtained was purified by silica gel column chromatography (eluent: methanol/dichloromethane). After purification, 10 mL of water/ethanol (EtOH) (1/1) solution of a 1 mol/L sodium hydroxide was added, and the mixture was stirred for 1 hour and washed with ethanol, and therefore 31 mg of a compound (1-D) was obtained. The compound (1-D) was identified by ESI-MS. The results are shown below.

ESI-MS: [M - H]⁻ = 939

In a 100 mL flask, 30 mg of the compound (1-D) and 10 mL of water were added, 10 mg of 1-[3-(dimethylamino) propyl]-3-ethylcarbodiimide (EDC) and 10 mg of N-hydroxysuccinimide were added thereto, and stirred for one hour. The resulting reaction solution was concentrated under reduced pressure, and the crude product thus obtained was purified by silica gel column chromatography (eluent: methanol/dichloromethane), and therefore 5 mg of a reference compound (1) was obtained. The reference compound (1) was identified by ESI-MS. The results are shown below.

ESI-MS: [M - H]⁻ = 1036

### Synthesis Example 2

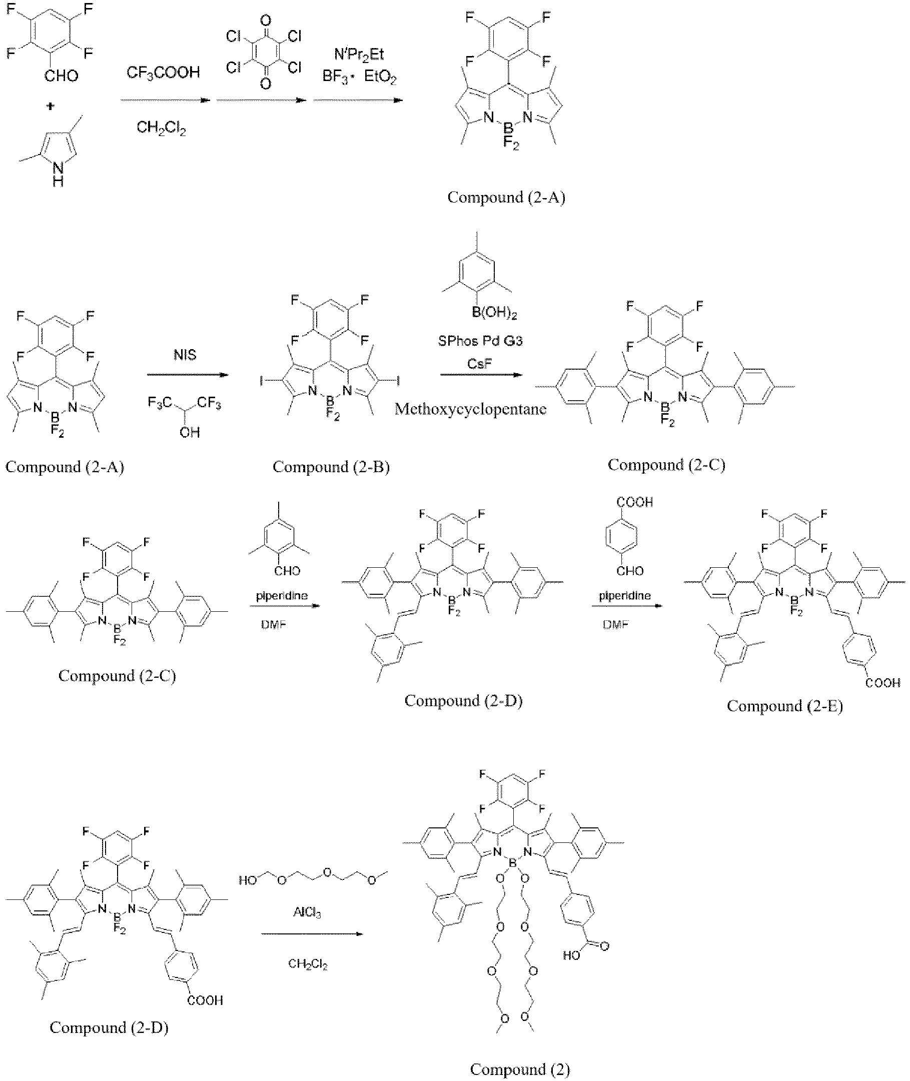

A compound (2-E) was synthesized in the same manner as in Synthesis Example 1 except that raw materials to be used were changed. The compound (2-E) was identified by ESI-MS. The results are shown below.

ESI-MS: [M - H]⁻ = 893

In a 50 mL flask, 100 mg of the compound (2-E) was weighed and dissolved in 3 mL of methylene chloride, 64 mg of aluminum trichloride was added thereto, and the mixture was heated under reflux for 30 minutes. Thereafter, the temperature was returned to room temperature, 1 mL of triethylene glycol monomethyl ether was added, and the reaction was carried out for 30 minutes. After completion of the reaction, after concentrating under reduced pressure, this crude product was purified by silica gel column chromatography (eluent: hexane/ethyl acetate) and then recrystallized from methanol/methylene chloride to obtain 32 mg of a compound (2). The compound (2) was identified by ESI-MS. The results are shown below.

ESI-MS: [M - H]⁻ = 1181

### Comparative compound

A comparative compound was synthesized according to the method disclosed in JP2012-513974A.

### <1> Evaluation of emission maximum wavelength and quantum yield of fluorescent dye solution

The fluorescent dyes (reference compound (1), compound (2), and comparative compound) synthesized as above were dissolved in phosphate buffered saline (PBS) solution, and the emission maximum wavelength and the quantum yield were evaluated. For the measurement of the emission maximum wavelength, the fluorescence spectrophotometer RF-5300 PC manufactured by Shimadzu Corporation was used, and the quantum yield was evaluated using the absolute PL (photoluminescence) quantum yield measuring device C9920-02 manufactured by Hamamatsu Photonics KK. An excitation wavelength was measured using a wavelength shorter than a maximum absorption wavelength of each compound by 50 nm. Evaluation standards of the emission maximum wavelength and the quantum yield are shown below. The results of the evaluation are shown in Table 1.

Emission maximum wavelength
S: 700 nm or longer
A: 680 nm or longer and shorter than 700 nm
B: 660 nm or longer and shorter than 680 nm
C: 640 nm or longer and shorter than 660 nm
D: 600 nm or longer and shorter than 640 nm
E: Shorter than 600 nm

Quantum yield
S: 0.85 or more
A: 0.8 or more and less than 0.85
B: 0.75 or more and less than 0.8
C: 0.7 or more and less than 0.75
D: 0.65 or more and less than 0.7
E: Less than 0.65

### <2> Evaluation of fluorescently labeled antibody

### Example 1: Evaluation of progesterone by competitive method

### Production of fluorescently labeled antibody

300 µL of 50 mmol/L 2-morpholinoethanesulfonic acid (MES, manufactured by Dojindo Laboratories) buffer solution (pH 6.0) was added to 300 µL of 0.03 mmol/L aqueous solution of the reference compound (1) synthesized as above. Thereafter, 300 µL of 0.5 mg/mL anti-progesterone monoclonal antibody (manufactured by GeneTex) was added, and the mixture was stirred at room temperature for 1.5 hours. Thereafter, gel column purification was carried out with Sephadex G-25 (manufactured by GE Healthcare), the mixture was freeze-dried to obtain a fluorescently labeled antibody.

### Production of substrate

150 µg of progesterone-BSA conjugate (manufactured by BIO-RAD) was added to and dissolved in 1 mL of a buffer solution (pH 5.2, 150 mmol/L NaCl) of a citrate concentration of 50 mmol/L, and therefore a solution of citrate buffer solution was obtained. A substrate of polymethyl methacrylate (PMMA) (ACRYPET (registered trademark) VH, manufactured by Mitsubishi Rayon Co., Ltd.) was prepared, a gold film having a thickness of 50 nm was vapor-deposited on one side by a sputtering method, the film was cut into a width of 7 mm so as to produce seven identical substrates. The solution of the prepared citrate buffer solution of the progesterone-BSA conjugate was spotted on the gold-deposited surface of the substrate and dried, and therefore a substrate on which the progesterone-BSA conjugate was immobilized was produced. A PBS solution (pH 7.4) containing TWEEN (registered trademark) 20 (polyoxyethylene (20) sorbitan monolaurate, manufactured by Wako Pure Chemical, Ltd.) by a concentration of 0.05% by mass was prepared in advance, and the prepared substrate was washed three times using 300 µL of this solution. After completing of washing, 300 µL of a PBS solution (pH 7.4) containing 1% by mass casein (manufactured by Thermo Scientific) was added to carry out blocking of an unadsorbed portion of the progesterone-BSA conjugate on the gold-vapor-deposited film, and allowed to stand at room temperature for 1 hour. After washing with the above-described washing solution, 300 µL of Immunoassay Stabilizer (manufactured by ABI) was added as a stabilizer, and the solution was allowed to stand at room temperature for 30 minutes, the solution was removed, moisture was completely removed by using a dryer, and therefore a substrate for evaluation was produced.

### Production of sensor chip

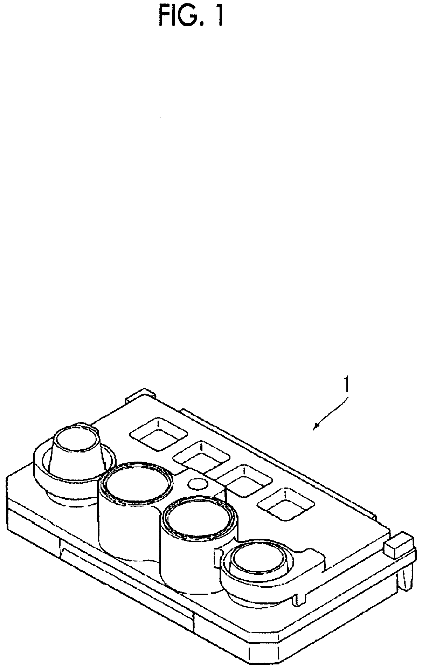

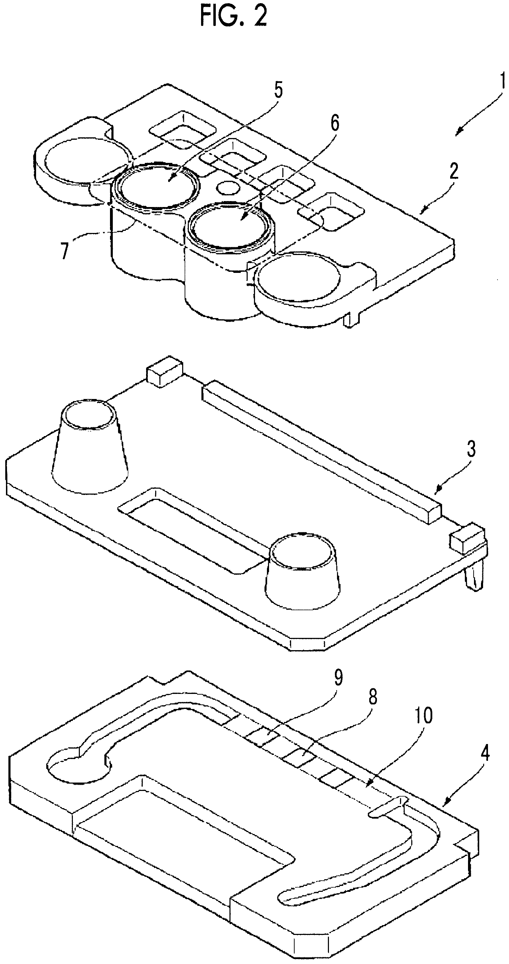

A flow passage-type sensor chip was produced so as to have the configuration of the second embodiment of JP2010-190880A. Figs. 1 and 2 are schematic views of the sensor chip. Fig. 1 is the schematic view of a sensor chip 1, and Fig. 2 is an exploded view of the sensor chip 1. The sensor chip 1 is configured of an upper member 2, an intermediate member 3, and a substrate 4. The upper member 2 has a first container 5 and a second container 6. The first container 5 and the second container 6 are collectively referred to as a container group 7. A flow passage 10 is formed in the substrate 4, and a detection region 8 and a reference region 9 are formed on the flow passage 10.

### Evaluation of progesterone

Using the fluorescently labeled antibody prepared above, measurement of progesterone was performed with IMMUNO AU10V (manufactured by Fujifilm Corporation). A sample containing 30.0 ng/mL progesterone was prepared. Next, an amount of the anti-progesterone antibody and an amount of progesterone at the time of producing the mixed solution of the fluorescently labeled anti-progesterone antibody prepared above and the sample, was adjusted to become 10:1, and the mixed solution of the fluorescently labeled antibody and the sample was prepared while stirring for 10 minutes. Next, the mixed solution of the fluorescently labeled antibody and the sample was spotted on the each flow passage-type sensor chip in which the substrate produced above was installed. After spotting, the mixed solution was allowed to flow down at a rate of 10 µL/min while performing pump suction and brought into contact with the gold film surface to which the progesterone-BSA conjugate was immobilized, and then the intensity of fluorescence was continuously measured for 1.5 minutes. A rate of increase in fluorescence intensity obtained in the substrate per unit time was obtained as a fluorescence signal value, and a relative fluorescence signal amount was compared. The calculation formulas and evaluation standard used for calculation are shown below. The results of the evaluation are shown in Table 1.

Relative fluorescence signal value = (fluorescence signal value when using each fluorescently labeled antibody)/(fluorescence signal value when using fluorescently labeled antibody produced in Comparative Example 1)

Relative fluorescence signal value
S: 2.0 or more
A: 1.8 or more and less than 2.0
B: 1.6 or more and less than 1.8
C: 1.4 or more and less than 1.6
D: 1.1 or more and less than 1.4
E: Less than 1.1

### Example 2: Evaluation of progesterone by competitive method

The compound (2) according to the invention was used instead of the reference compound (1) in the production of the fluorescently labeled antibody of Example 1. 300 µL of 50 mmol/L 2-morpholinoethanesulfonic acid (MES, manufactured by Dojindo Laboratories) buffer solution (pH 6.0) was added to 150 µL of 0.06 mmol/L solution, and 150 µL of 0.06 mmol/L EDC aqueous solution (1-[3-(dimethylamino)propyl]-3-ethylcarbodiimide) was added thereto. Thereafter, 300 µL of 0.5 mg/mL anti-progesterone monoclonal antibody (manufactured by GeneTex) was added, and the mixture was stirred at room temperature for 1.5 hours. Thereafter, gel column purification was carried out with Sephadex G-25 (manufactured by GE Healthcare), the mixture was freeze-dried to obtain a fluorescently labeled antibody. Evaluation was carried out by the method described in Example 1 using the obtained fluorescently labeled antibody. The results of the evaluation are shown in Table 1.

### Comparative Example 1: Evaluation of progesterone by competitive method

Evaluation was carried out in the same manner as in Example 2 except that the comparative compound was used instead of the compound (2). The results of the evaluation are shown in Table 1.

### Example 3: Evaluation of TSH by sandwich method

### Production of fluorescently labeled antibody

300 µL of 50 mmol/L 2-morpholinoethanesulfonic acid (MES, manufactured by Dojindo Laboratories) buffer solution (pH 6.0) was added to 300 µL of 0.03 mmol/L aqueous solution of the reference compound (1) synthesized as above. Thereafter, 300 µL of 0.5 mg/mL anti-TSH monoclonal antibody (manufactured by Meridian life science: Anti-TSH Mab MAT04-410) was added, and the mixture was stirred at room temperature for 1.5 hours. Thereafter, gel column purification was carried out with Sephadex G-25 (manufactured by GE Healthcare), the mixture was freeze-dried to obtain a fluorescently labeled antibody.

### Production of substrate

A substrate of polymethyl methacrylate (PMMA) (ACRYPET (registered trademark) VH, manufactured by Mitsubishi Rayon Co., Ltd.) was prepared, a gold film having a thickness of 45 nm was vapor-deposited on one side by a sputtering method, the film was cut into a width of 7 mm so as to produce seven identical substrates. On the gold-deposited surface of this substrate, a solution having a concentration of 1 mm mol/L, which is prepared by mixing 1-Undecanthiol (manufactured by Wako Pure Chemical Industries, Ltd., 323-78512), and 11-Hydroxy-1-undecanethiol which is an alkanethiol having a hydroxyl group at the terminal (manufactured by Dojindo Laboratories Inc., H337) at a weight ratio of 99:1, was added to the gold-deposited surface, the mixture was allowed to stand for 16 hours, and therefore a self-assembled film was formed. Thereafter, the mixed solution was removed, washing was performed three times with 100 µL of hexane and once with 100 µL of ultrapure water, and then drying was performed. A solution (concentration: 10 µg/mL in 150 mmol/L NaCl) containing the prepared anti-TSH monoclonal antibody 1 (manufactured by Medix, 5409) was spotted on the self-assembled film formed as described above, allowed to stand for 1 hour, and then dried, and therefore a substrate on which the anti-TSH antibody was immobilized was produced. A PBS solution (pH 7.4) containing TWEEN (registered trademark) 20 (polyoxyethylene (20) sorbitan monolaurate, manufactured by Wako Pure Chemical, Ltd.) by a concentration of 0.05% by mass was prepared in advance, and the prepared substrate was washed three times using 300 µL of this solution. After completing of washing, 300 µL of a PBS solution (pH 7.4) containing 1% by mass casein (manufactured by Thermo Scientific) was added to carry out blocking of an unadsorbed portion of the anti-TSH antibody on the gold film, and allowed to stand at room temperature for 1 hour. After washing with the above-described washing solution, 300 µL of Immunoassay Stabilizer (manufactured by ABI) was added as a stabilizer, and the solution was allowed to stand at room temperature for 30 minutes, the solution was removed, moisture was completely removed by using a dryer, and therefore a substrate for evaluation was produced.

### Production of sensor chip

A flow passage-type sensor chip was produced so as to have the configuration of the second embodiment of JP2010-190880A. Figs. 1 and 2 are schematic views of the sensor chip. Fig. 1 is the schematic view of a sensor chip 1, and Fig. 2 is an exploded view of the sensor chip 1. The sensor chip 1 is configured of an upper member 2, an intermediate member 3, and a substrate 4. The upper member 2 has a first container 5 and a second container 6. The first container 5 and the second container 6 are collectively referred to as a container group 7. A flow passage 10 is formed in the substrate 4, and a detection region 8 and a reference region 9 are formed on the flow passage 10.

### Preparation of test sample

As a test sample, canine serum was used. As the canine serum, serum of Toyo Vehicle Dogs purchased from KITAYAMA LABES CO., LTD. was prepared.

### Evaluation of TSH

Using the fluorescently labeled antibody prepared above, measurement of TSH was performed with IMMUNO AU10V (manufactured by Fujifilm Corporation). A mixed solution of 100 µL of the test sample (canine serum) prepared above and the fluorescently labeled anti-TSH antibody was prepared, and the mixed solution was stirred for 10 minutes. Next, the mixed solution of the fluorescently labeled antibody and the test sample was spotted on the each flow passage-type sensor chip in which the substrate produced above was installed. After spotting, the mixed solution was allowed to flow down at a rate of 10 µL/min while performing pump suction and brought into contact with the gold film surface to which the anti-TSH antibody was immobilized, and then the intensity of fluorescence was continuously measured for 1.5 minutes. A rate of increase in fluorescence intensity obtained in the substrate per unit time was obtained as a fluorescence signal value, and a relative fluorescence signal amount was compared. The calculation formulas and evaluation standard used for calculation are shown below. The results of the evaluation are shown in Table 1.

Relative fluorescence signal value = (fluorescence signal value when using each fluorescently labeled antibody)/(fluorescence signal value when using fluorescently labeled antibody produced in Comparative Example 2)

Relative fluorescence signal value
S: 2.0 or more
A: 1.8 or more and less than 2.0
B: 1.6 or more and less than 1.8
C: 1.4 or more and less than 1.6
D: 1.1 or more and less than 1.4
E: Less than 1.1

### Example 4: Evaluation of TSH by sandwich method

The compound (2) according to the invention was used instead of the reference compound (1) in the production of the fluorescently labeled antibody of Example 3. 300 µL of 50 mmol/L 2-morpholinoethanesulfonic acid (MES, manufactured by Dojindo Laboratories) buffer solution (pH 6.0) was added to 150 µL of 0.06 mmol/L aqueous solution, and 150 µL of 0.06 mmol/L EDC aqueous solution (1-[3-(dimethylamino)propyl]-3-ethylcarbodiimide) was added thereto. Thereafter, 300 µL of 0.5 mg/mL anti-TSH monoclonal antibody (manufactured by Meridian life science: Anti-TSH Mab MAT04-410) was added, and the mixture was stirred at room temperature for 1.5 hours. Thereafter, gel column purification was carried out with Sephadex G-25 (manufactured by GE Healthcare), the mixture was freeze-dried to obtain a fluorescently labeled antibody. Evaluation was carried out by the method described in Example 1 using the obtained fluorescently labeled antibody. The results of the evaluation are shown in Table 1.

### Comparative Example 2: Evaluation of TSH by sandwich method

Evaluation was carried out in the same manner as in Example 4 except that the comparative compound was used instead of the compound (2) according to the invention. The results of the evaluation are shown in Table 1.

**[Table 1]**

| | Compound | Substance to be measured | Emission maximum wavelength | Quantum yield | Relative fluorescence signal |
|---|---|---|---|---|---|
| Example 1 | Compound (1) | Progesterone | A | A | A |
| Example 2 | Compound (2) | Progesterone | s | s | s |
| Example 3 | Compound (1) | TSH | A | A | A |
| Example 4 | Compound (2) | TSH | s | s | s |
| Comparative Example 1 | Comparative compound | Progesterone | D | E | E |
| Comparative Example 2 | Comparative compound | TSH | D | E | E |

In the case of using the reference compound (1) or the compound (2) according to the embodiment of the invention, it is understood that the quantum yield is high, the relative fluorescence signal is higher than that of the comparative compound, and the substance to be measured can be detected with high-sensitivity. Compound (2) is superior to reference compound (1). Explanation of References

1: sensor chip
2: upper member
3: intermediate member
4: substrate
5: first container
6: second container
7: container group
8: detection region
9: reference region
10: flow passage

## Claims

1. A kit for measuring a substance to be measured, comprising:
a compound represented by Formula (1) that binds to a first binding substance capable of binding to the substance to be measured; and
a substrate that includes, on a metal film, a detection region having a second binding substance capable of binding to any one of the substance to be measured or the first binding substance, 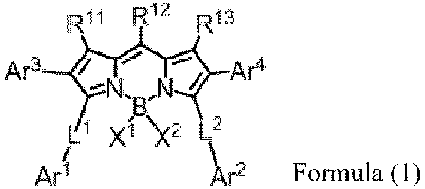
in the formula, R¹¹, , and R¹³ each represent an alkyl group, and these groups may have a substituent; R¹² represents a phenyl group, and this group may have a substituent; L¹ and L² each represent Formula (L-1); Ar¹, Ar², Ar³, and Ar⁴ each represent an aryl group, and these groups independently may have a substituent; and X¹ and X² each independently represent a halogen atom or an alkoxy group, and these groups may have a substituent, provided that at least one of R¹¹, R¹², R¹³, Ar¹, Ar², Ar³, Ar⁴, X¹, or X² has a hydrophilic part, wherein the hydrophilic part has a polyethylene glycol structure, a betaine structure, an amino acid structure, or a sulfonic acid group, and at least one of R¹¹, R¹², R¹³, Ar¹, Ar², Ar³, Ar⁴, X¹, or X² has a capture part capable of forming a covalent bond with a biological molecule, wherein the capture part is a carboxyl group, an active ester, an isothiocyanate, or a maleimide, 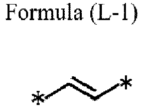
and further wherein at least one of Ar¹ or Ar² in Formula (1) is Formula (Ar-1), and at least one of Ar³ or Ar⁴ in Formula (1) is Formula (Ar-1), 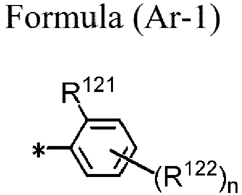 in the formula, R¹²¹ and R¹²² each independently represent a halogen atom, an alkyl group, or an alkoxy group, provided that R¹²¹ and R¹²² each may have a hydrophilic part or a capture part capable of forming a covalent bond with a biological molecule, wherein the hydrophilic part has a polyethylene glycol structure, a betaine structure, an amino acid structure, or a sulfonic acid group, and wherein the capture part is a carboxyl group, an active ester, an isothiocyanate, or a maleimide, n represents an integer of 0 to 4, and in a case where n is 2 or more, a plurality of R¹²²'s may be the same or different from each other, the substituents being selected from the group consisting of a sulfamoyl group, a cyano group, an isocyano group, a thiocyanato group, an isothiocyanato group, a nitro group, a nitrosyl group, a halogen atom, a hydroxy group, an amino group, a mercapto group, an amide group, an alkoxy group, an aryloxy group, an alkylthio group, an arylthio group, a carbamoyl group, an acyl group, an aldehyde group, a carbonyl group, an aryl group, an alkyl group, an alkyl group substituted with a halogen atom, an ethenyl group, an ethynyl group, a silyl group and a trialkylsilyl group.

2. The kit according to claim 1,
wherein an emission maximum wavelength of the compound represented by Formula (1) is 600 nm or longer.

3. The kit according to claim 1 or 2,
wherein the metal film is a metal film including gold.

4. The kit according to any one of claims 1 to 3,
wherein the first binding substance is an antibody.

5. The kit according to any one of claims 1 to 4,
wherein the detection region has the second binding substance capable of binding to the substance to be measured, and
the second binding substance is an antibody.

6. A fluorescent labeling agent, comprising:
at least one kind of a compound represented by Formula (1), 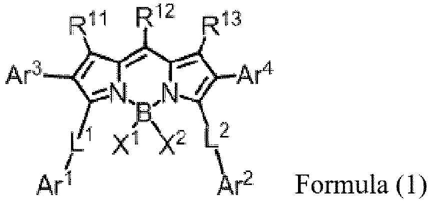
in the formula, R¹¹, and R¹³ each represent an alkyl group, and these groups may have a substituent R¹² represents a phenyl group, and this group may have a substituent;; L¹ and L² each represent Formula (L-1); Ar¹, Ar², Ar³, and Ar⁴ each represent an aryl group, and these groups independently may have a substituent; and X¹ and X² each independently represent a halogen atom or an alkoxy group, and these groups may have a substituent, provided that at least one of R¹¹, R¹², R¹³, Ar¹, Ar², Ar³, Ar⁴, X¹, or X² has a hydrophilic part, wherein the hydrophilic part has a polyethylene glycol structure, a betaine structure, an amino acid structure, or a sulfonic acid group, and at least one of R¹¹, R¹², R¹³, Ar¹, Ar², Ar³, Ar⁴, X¹, or X² has a capture part capable of forming a covalent bond with a biological molecule, wherein the capture part is a carboxyl group, an active ester, an isothiocyanate, or a maleimide, 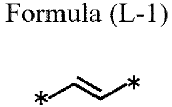 and further wherein at least one of Ar¹ or Ar² in Formula (1) is Formula (Ar-1), and at least one of Ar³ or Ar⁴ in Formula (1) is Formula (Ar-1), 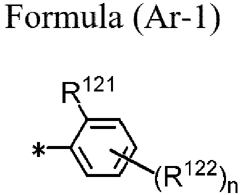
in the formula, R¹²¹ and R¹²² each independently represent a halogen atom, an alkyl group, or an alkoxy group, provided that R¹²¹ and R¹²² each may have a hydrophilic part or a capture part capable of forming a covalent bond with a biological molecule, wherein the hydrophilic part has a polyethylene glycol structure, a betaine structure, an amino acid structure, or a sulfonic acid group, and wherein the capture part is a carboxyl group, an active ester, an isothiocyanate, or a maleimide, n represents an integer of 0 to 4, and in a case where n is 2 or more, a plurality of R¹²²'s may be the same or different from each other, the substituents being selected from the group consisting of a sulfamoyl group, a cyano group, an isocyano group, a thiocyanato group, an isothiocyanato group, a nitro group, a nitrosyl group, a halogen atom, a hydroxy group, an amino group, a mercapto group, an amide group, an alkoxy group, an aryloxy group, an alkylthio group, an arylthio group, a carbamoyl group, an acyl group, an aldehyde group, a carbonyl group, an aryl group, an alkyl group, an alkyl group substituted with a halogen atom, an ethenyl group, an ethynyl group, a silyl group and a trialkylsilyl group.

7. The fluorescent labeling agent according to claim 6, wherein the fluorescent labeling agent is bound to an antibody.

8. The fluorescent labeling agent according to claim 7, wherein the fluorescent labeling agent is bound to the antibody via an amide bond.

## Patentansprüche

1. Kit zum Messen einer zu messenden Substanz, aufweisend:
eine Verbindung, die repräsentiert wird durch Formel (1), die an eine erste Bindungssubstanz, die in der Lage ist, an die zu messende Substanz zu binden, bindet; und
ein Substrat, das auf einem Metallfilm einen Nachweisbereich mit einer zweiten Bindungssubstanz, die in der Lage ist, an die zu messende Substanz oder an die erste Bindungssubstanz zu binden, enthält, 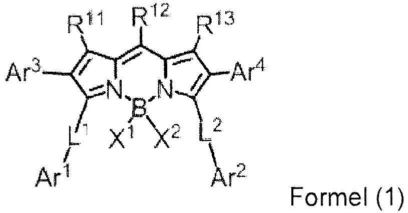 wobei in der Formel R¹¹ und R¹³ jeweils eine Alkylgruppe repräsentieren, und diese Gruppen einen Substituenten haben können; R¹² eine Phenylgruppe repräsentiert, und diese Gruppe einen Substituenten haben kann; L¹ und L² jeweils Formel (L-1) repräsentieren; Ar¹, Ar², Ar³ und Ar⁴ jeweils eine Arylgruppe repräsentieren, und diese Gruppen unabhängig voneinander einen Substituenten haben können; und X¹ und X² jeweils unabhängig ein Halogenatom oder eine Alkoxygruppe repräsentieren , und diese Gruppen einen Substituenten haben können, mit der Maßgabe, dass mindestens eine der Gruppen R¹¹, R¹², R¹³, Ar¹, Ar², Ar³, Ar⁴, X¹ oder X² einen hydrophilen Teil hat, wobei der hydrophile Teil eine Polyethylenglykol- Struktur, eine Betain-Struktur, eine Aminosäure-Struktur oder eine Sulfonsäuregruppe besitzt, und mindestens eine der Gruppen R¹¹, R¹², R¹³, Ar¹, Ar², Ar³, Ar⁴, X¹ oder X² einen Einfangteil besitzt, der in der Lage ist, mit einem biologischen Molekül eine kovalente Bindung zu bilden, wobei der Einfangteil eine Carboxylgruppe, ein aktiver Ester, ein Isothiocyanat oder ein Maleimid ist, 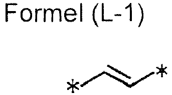 und wobei außerdem mindestens eine der Gruppen Ar¹ oder Ar² in Formel (1) die Formel (Ar-1) ist, und mindestens eine der Gruppen Ar³ oder Ar⁴ in Formel (1) die Formel (Ar-1) ist, 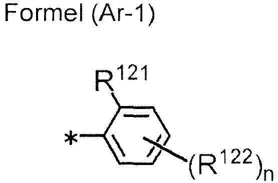 wobei in der Formel R¹²¹ und R¹²² jeweils unabhängig ein Halogenatom, eine Alkylgruppe oder eine Alkoxygruppe repräsentieren mit der Maßgabe, dass jede der Gruppen R¹²¹ und R¹²² einen hydrophilen Teil oder einen Einfangteil besitzen kann, der in der Lage ist, mit einem biologischen Molekül eine kovalente Bindung zu bilden, wobei der hydrophile Teil eine Polyethylenglykol-Struktur, eine Betain-Struktur, eine Aminosäure-Struktur oder eine Sulfonsäuregruppe besitzt, und wobei der Einfangteil eine Carboxylgruppe, ein aktiver Ester, ein Isothiocyanat oder ein Maleimid ist, n eine ganze Zahl von 0 bis 4 repräsentiert, und in einem Fall, in dem n 2 oder mehr beträgt, mehrere R¹²² gleich oder verschieden voneinander sein können, wobei die Substituenten ausgewählt sind aus der Gruppe, die aus einer Sulfamoylgruppe, einer Cyanogruppe, einer Isocyanogruppe, einer Thiocyanatogruppe, einer Isothiocyanatogruppe, einer Nitrogruppe, einer Nitrosylgruppe, einem Halogenatom, einer Hydroxygruppe, einer Aminogruppe, einer Mercaptogruppe, einer Amidgruppe, einer Alkoxygruppe, einer Aryloxygruppe, einer Alkylthiogruppe, einer Arylthiogruppe, einer Carbamoylgruppe, einer Acylgruppe, einer Aldehydgruppe, einer Carbonylgruppe, einer Arylgruppe, einer Alkylgruppe, einer mit einem Halogenatom substituierten Alkylgruppe, einer Ethenylgruppe, einer Ethynylgruppe, einer Silylgruppe und einer Trialkylsilylgruppe besteht.

2. Kit nach Anspruch 1,
wobei eine Emissionsmaximum-Wellenlänge der durch Formel (1) repräsentierten Verbindung 600 nm oder länger ist.

3. Kit nach Anspruch 1 oder 2,
wobei der Metallfilm ein Gold enthaltender Metallfilm ist.

4. Kit nach einem der Ansprüche 1 bis 3,
wobei die erste Bindungssubstanz ein Antikörper ist.

5. Kit nach einem der Ansprüche 1 bis 4,
wobei der Nachweisbereich die zweite Bindungssubstanz aufweist, die in der Lage ist, an die zu messende Substanz zu binden, und
die zweite Bindungssubstanz ein Antikörper ist.

6. Fluoreszierendes Markierungsmittel aufweisend:
mindestens eine Art von Verbindung, die repräsentiert wird durch Formel (1), 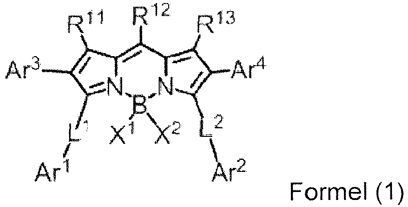 wobei in der Formel R¹¹ und R¹³ jeweils eine Alkylgruppe repräsentieren, und diese Gruppen einen Substituenten haben können; R¹² eine Phenylgruppe repräsentiert, und diese Gruppe einen Substituenten haben kann; L¹ und L² jeweils Formel (L-1) repräsentieren; Ar¹, Ar², Ar³ und Ar⁴ jeweils eine Arylgruppe repräsentieren, und diese Gruppen unabhängig voneinander einen Substituenten haben können; und X¹ und X² jeweils unabhängig ein Halogenatom oder eine Alkoxygruppe repräsentieren , und diese Gruppen einen Substituenten haben können, mit der Maßgabe, dass mindestens eine der Gruppen R¹¹, R¹², R¹³, Ar¹, Ar², Ar³, Ar⁴, X¹ oder X² einen hydrophilen Teil hat, wobei der hydrophile Teil eine Polyethylenglykol- Struktur, eine Betain-Struktur, eine Aminosäure-Struktur oder eine Sulfonsäuregruppe besitzt, und mindestens eine der Gruppen R¹¹, R¹², R¹³, Ar¹, Ar², Ar³, Ar⁴, X¹ oder X² einen Einfangteil besitzt, der in der Lage ist, mit einem biologischen Molekül eine kovalente Bindung zu bilden, wobei der Einfangteil eine Carboxylgruppe, ein aktiver Ester, ein Isothiocyanat oder ein Maleimid ist, 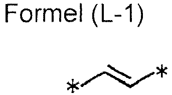 und wobei außerdem mindestens eine der Gruppen Ar¹ oder Ar² in Formel (1) die Formel (Ar-1) ist, und mindestens eine der Gruppen Ar³ oder Ar⁴ in Formel (1) die Formel (Ar-1) ist, 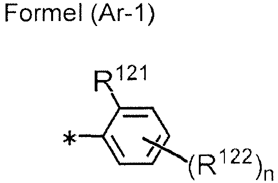 wobei in der Formel R¹²¹ und R¹²² jeweils unabhängig ein Halogenatom, eine Alkylgruppe oder eine Alkoxygruppe repräsentieren mit der Maßgabe, dass jede der Gruppen R¹²¹ und R¹²² einen hydrophilen Teil oder einen Einfangteil besitzen kann, der in der Lage ist, mit einem biologischen Molekül eine kovalente Bindung zu bilden, wobei der hydrophile Teil eine Polyethylenglykol-Struktur, eine Betain-Struktur, eine Aminosäure-Struktur oder eine Sulfonsäuregruppe besitzt, und wobei der Einfangteil eine Carboxylgruppe, ein aktiver Ester, ein Isothiozyanat oder ein Maleimid ist, n eine ganze Zahl von 0 bis 4 repräsentiert, und in einem Fall, in dem n 2 oder mehr beträgt, mehrere R¹²² gleich oder verschieden voneinander sein können, wobei die Substituenten ausgewählt sind aus der Gruppe, die aus einer Sulfamoylgruppe, einer Cyanogruppe, einer Isocyanogruppe, einer Thiocyanatogruppe, einer Isothiocyanatogruppe, einer Nitrogruppe, einer Nitrosylgruppe, einem Halogenatom, einer Hydroxygruppe, einer Aminogruppe, einer Mercaptogruppe, einer Amidgruppe, einer Alkoxygruppe, einer Aryloxygruppe, einer Alkylthiogruppe, einer Arylthiogruppe, einer Carbamoylgruppe, einer Acylgruppe, einer Aldehydgruppe, einer Carbonylgruppe, einer Arylgruppe, einer Alkylgruppe, einer mit einem Halogenatom substituierten Alkylgruppe, einer Ethenylgruppe, einer Ethynylgruppe, einer Silylgruppe und einer Trialkylsilylgruppe besteht.

7. Fluoreszierendes Markierungsmittel nach Anspruch 6, wobei das fluoreszierende Markierungsmittel an einen Antikörper gebunden ist.

8. Fluoreszierendes Markierungsmittel nach Anspruch 7, wobei das fluoreszierende Markierungsmittel über eine Amidbindung an den Antikörper gebunden ist.

## Revendications

1. Kit destiné à mesurer une substance devant être mesurée, comprenant :
un composé représenté par la formule (1), lequel se lie à une première substance de liaison capable de se lier à la substance devant être mesurée, et
un substrat, lequel inclut, sur un film métallique, une région de détection présentant une seconde substance de liaison capable de se lier à l'une quelconque substance devant être mesurée ou la première substance de liaison, 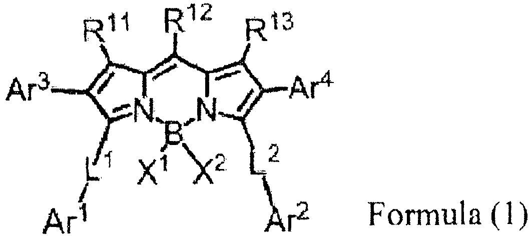
dans la formule, R¹¹ et R¹³ représentent chacun un groupe alkyle, et ces groupes peuvent présenter un substituant ; R¹² représente un groupe phényle, et ce groupe peut présenter un substituant ; L¹ et L² représentent chacun la formule (L-1) ; Ar¹, Ar², Ar³, et Ar⁴, représentent chacun un groupe aryle, et ces groupes peuvent présenter indépendamment un substituant, et X¹ et X² représentent chacun indépendamment un atome d'halogène ou un groupe alkoxy, et ces groupes peuvent présenter un substituant, à condition qu'au moins un parmi R¹¹, R¹², R¹³, Ar¹, Ar²,. Ar³, Ar⁴, X¹ ou X² présente une partie hydrophile, dans lequel la partie hydrophile présente une structure de polyéthylèneglycol, une structure de bétaïne, une structure d'aminoacide, ou un groupe acide sulfonique, et au moins un parmi R¹¹, R¹², R¹³, Ar¹, Ar²,. Ar³, Ar⁴, X¹ ou X² présente une partie de capture capable de former une liaison covalente avec une molécule biologique ; dans lequel la partie de capture est un groupe carboxyle, un ester actif, un isothiocyanate, ou un maléimide, 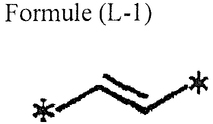
et dans lequel en outre, au moins un parmi Ar¹ ou Ar² dans la formule (1) est la formule (Ar-1), et au moins un parmi Ar³ ou Ar⁴ dans la formule (1) est la formule (Ar-1) ; 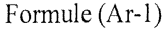 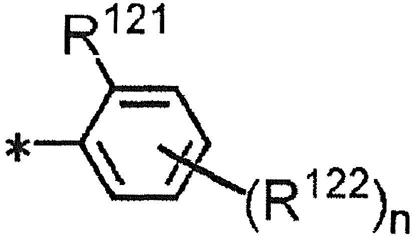
dans la formule, R¹²¹ et R¹²² représentent chacun indépendamment un atome d'halogène, un groupe alkyle ou un groupe alkoxy, à condition que R¹²¹ et R¹²² puissent présenter chacun une partie hydrophile ou une partie de capture capable de former une liaison covalente avec une molécule biologique ; dans lequel la partie hydrophile présente une structure de polyéthylèneglycol, une structure de bétaïne, une structure d'aminoacide, ou un groupe acide sulfonique, et dans lequel la partie de capture est un groupe carboxyle, un ester actif, un isothiocyanate, ou un maléimide ; n représente un entier allant de 0 à 4, et dans un cas où n est supérieur ou égal à 2, une pluralité de R¹²² peuvent être identiques ou différents entre eux, les substituants pouvant être sélectionnés parmi le groupe consistant en un groupe sulfamoyle, un groupe cyano, un groupe isocyano, un groupe thiocyanato, un groupe isothiocyanato, un groupe nitro, un groupe nitrosyle, un atome d'halogène, un groupe hydroxy, un groupe amino, un groupe mercapto, un groupe amide, un groupe alkoxy, un groupe aryloxy, un groupe alkylthio, un groupe arylthio, un groupe carbomoyle, un groupe acyle, un groupe aldéhyde, un groupe carbonyle, un groupe aryle, un groupe alkyle, un groupe alkyle substitué avec un atome d'halogène, un groupe éthényle, un groupe éthynyle, un groupe silyle, et un groupe trialkylsilyle.

2. Kit selon la revendication 1,
dans lequel une longueur d'onde maximale d'émission du composé représenté par la formule (1) est supérieure ou égale à 600 nm.

3. Kit selon la revendication 1 ou 2,
dans lequel le film métallique est un film métallique incluant de l'or.

4. Kit selon l'une quelconque des revendications 1 à 3,
dans lequel la première substance de liaison est un anticorps.

5. Kit selon l'une quelconque des revendications 1 à 4,
dans lequel la région de détection présente la seconde substance de liaison capable de se lier à la substance devant être mesurée, et
la seconde substance de liaison est un anticorps.

6. Agent d'étiquetage fluorescent, comprenant :
au moins un type d'un composé représenté par la formule (1) 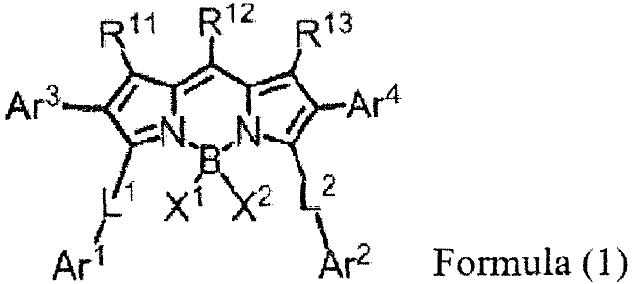
dans la formule, R¹¹ et R¹³ représentent chacun un groupe alkyle, et ces groupes peuvent présenter un substituant ; R¹² représente un groupe phényle, et ce groupe peut présenter un substituant ; L¹ et L² représentent chacun la formule (L-1) ; Ar¹, Ar², Ar³, et Ar⁴, représentent chacun un groupe aryle, et ces groupes peuvent présenter indépendamment un substituant, et X¹ et X² représentent chacun indépendamment un atome d'halogène ou un groupe alkoxy, et ces groupes peuvent présenter un substituant, à condition qu'au moins un parmi R¹¹, R¹², R¹³, Ar¹, Ar², Ar³, Ar⁴, X¹ ou X² présente une partie hydrophile, dans lequel la partie hydrophile présente une structure de polyéthylèneglycol, une structure de bétaïne, une structure d'aminoacide, ou un groupe acide sulfonique, et au moins un parmi R¹¹, R¹², R¹³, Ar¹, Ar², Ar³, Ar⁴, X¹ ou X² présente une partie de capture capable de former une liaison covalente avec une molécule biologique ; dans lequel la partie de capture est un groupe carboxyle, un ester actif, un isothiocyanate, ou un maléimide, 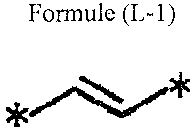 et dans lequel en outre, au moins un parmi Ar¹ ou Ar² dans la formule (1) est la formule (Ar-1), et au moins un parmi Ar³ ou Ar⁴ dans la formule (1) est la formule (Ar-1) ; 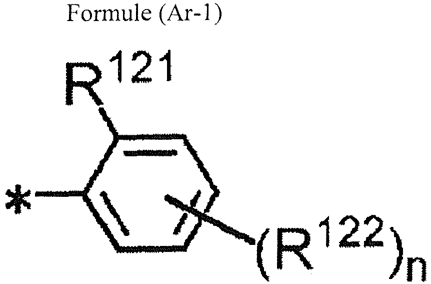
dans la formule, R¹²¹ et R¹²² représentent chacun indépendamment un atome d'halogène, un groupe alkyle, ou un groupe alkoxy, à condition que R¹²¹ et R¹²² puissent présenter chacun une partie hydrophile ou une partie de capture capable de former une liaison covalente avec une molécule biologique ; dans lequel la partie hydrophile présente une structure de polyéthylèneglycol, une structure de bétaïne, une structure d'aminoacide, ou un groupe acide sulfonique, et dans lequel la partie de capture est un groupe carboxyle, un ester actif, un isothiocyanate, ou un maléimide ; n représente un entier allant de 0 à 4, et dans un cas où n est supérieur ou égal à 2, une pluralité de R¹²² peuvent être identiques ou différents entre eux, les substituants pouvant être sélectionnés parmi le groupe consistant en un groupe sulfamoyle, un groupe cyano, un groupe isocyano, un groupe thiocyanato, un groupe isothiocyanato, un groupe nitro, un groupe nitrosyle, un atome d'halogène, un groupe hydroxy, un groupe amino, un groupe mercapto, un groupe amide, un groupe alkoxy, un groupe aryloxy, un groupe alkylthio, un groupe arylthio, un groupe carbomoyle, un groupe acyle, un groupe aldéhyde, un groupe carbonyle, un groupe aryle, un groupe alkyle, un groupe alkyle substitué avec un atome d'halogène, un groupe éthényle, un groupe éthynyle, un groupe silyle, et un groupe trialkylsilyle.

7. Agent d'étiquetage fluorescent selon la revendication 6, dans lequel l'agent d'étiquetage fluorescent est lié à un anticorps.

8. Agent d'étiquetage fluorescent selon la revendication 7, dans lequel l'agent d'étiquetage fluorescent est lié à l'anticorps via une liaison amide.
